# EUROPÄISCHE PATENTSCHRIFT

(11) **EP 2 726 122 B1**
(45) Veröffentlichungstag und Bekanntmachung des Hinweises auf die Patenterteilung: **21.04.2021**
(21) Anmeldenummer: 12725320.1
(22) Anmeldetag: 31.05.2012
(51) Int. Cl.: A61M 1/28

(54) **DIALYSEMASCHINE ZUR EINSTELLUNG EINES KONTINUIERLICHEN DIALYSATVOLUMENSTROMES**
DIALYSIS MACHINE FOR SETTING A CONTINUOUS DIALYSATE VOLUME FLOW RATE
MACHINE DE DIALYSE POUR RÉGLER UN DÉBIT VOLUMÉTRIQUE CONTINU DE DIALYSAT

(30) Priorität: 03.06.2011 DE 102011103325; 03.06.2011 US 201161492952 P
(43) Veröffentlichungstag der Anmeldung: 07.05.2014
(73) Patentinhaber: Fresenius Medical Care Deutschland GmbH, 61352 Bad Homburg (DE)
(72) Erfinder: HEDMANN, Frank, 97332 Volkach (DE); GRIESMANN, Erik, 97422 Schweinfurt (DE); WICH-HEITER, Joachim, 97273 Kürnach (DE); SEBESTA, Sven, 97421 Schweinfurt (DE)
(74) Vertreter: Laufhütte, Dieter
(86) Internationale Anmeldenummer: PCT/EP2012/002322
(87) Internationale Veröffentlichungsnummer: WO 2012/163537

(56) Entgegenhaltungen:
- US-A- 4 618 343
- US-A1- 2010 168 652
- US-B1- 6 503 062

## Beschreibung

Die Erfindung betrifft eine Dialysemaschine mit einer Steuerung zur Einstellung eines kontinuierlichen Dialysatvolumenstroms. Bei der Dialysemaschine kann es sich dabei insbesondere um eine Peritonealdialysemaschine handeln.

Bei der automatischen Peritonealdialyse steuert und überwacht eine Dialysemaschine die Einführung des frischen Dialysats in die Bauchhöhle und das Auslassen des verbrauchten Dialysats. Eine solche Dialysemaschine, auch Cycler genannt, befüllt und entleert die Bauchhöhle dabei üblicherweise mehrmals über Nacht, d. h. während der Patient schläft.

Die US 2010/0168652 beschreibt ein Verfahren zur Einstellung eines kontinuierlichen Dialysatvolumenstroms, wobei es sich bei den verwendeten Pumpen um piezoelektrische Pumpen handelt, die mittels einer Spannungsquelle betrieben werden. Dabei erzeugt ein Piezoelement ein elektrisches Signal (Strom oder Spannung), das dem Grad und der Richtung seiner Biegung entspricht. Das elektrische Signal wird an einen Prozessor weitergeleitet und in das jeweilige Volumen der Lösung umgewandelt.

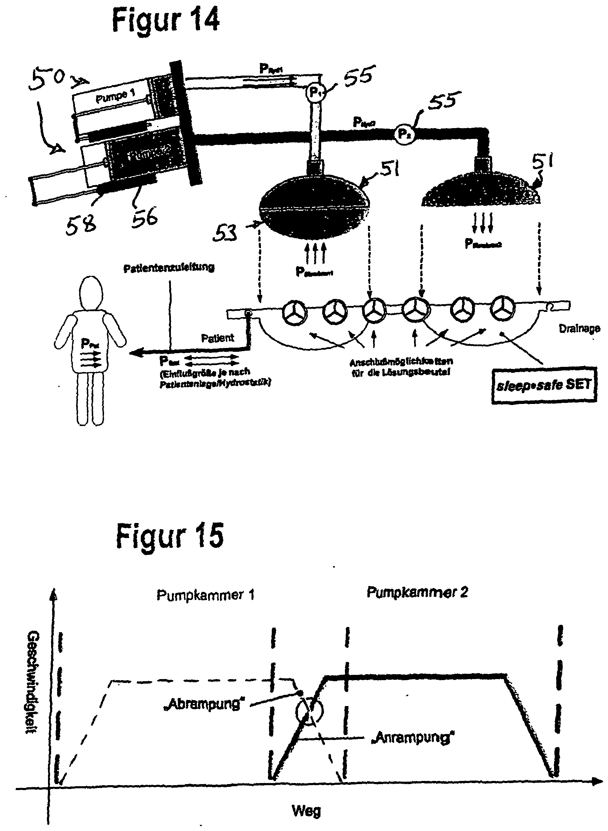

Eine weitere Peritonealdialysemaschine nach dem Stand der Technik ist beispielhaft in Figur 14 dargestellt. Wie hier dargestellt ist, werden üblicherweise zwei Pumpen 50 eingesetzt, die als Pumpaktoren 51 Membranpumpen aufweisen. Diese wirken auf Pumpkammern, mit welchen Dialysat aus entsprechend vorhandenen Dialysatbeuteln in die Bauchhöhle eines Patienten gepumpt bzw. verbrauchtes Dialysat aus der Bauchhöhle des Patienten ausgelassen wird. Um trotz der diskontinuierlich arbeitenden Membranpumpe einen konstanten Dialysatvolumenstrom zu erreichen, wird der hydraulische Druck P_{Hyd} in den Hydraulikleitungen der Membranpumpen bestimmt. Für den Fall, daß die Membranpumpen pneumatisch angetrieben werden, wird der entsprechende pneumatische Druck in den Leitungen bestimmt. Um eine Drucküberwachung zu gewährleisten, müssen die mittels der Drucksensoren 55 gemessenen Drücke P_{Hyd} um einige Einflusswerte kompensiert werden. Hierbei handelt es sich zum einen um den jeweiligen Membrandruck P_{Membran}, d. h. den Gegendruck, der auf den gemessenen Hydraulikdruck P_{Hyd} durch die Auslenkung und Eigenspannung der Membran hervorgerufen wird. Mit zunehmender Auslenkung nimmt die Membranspannung überproportional zu, womit eine konstruktiv bedingte Geschwindigkeitsreaktion einhergeht. Dieser Gegendruck hängt von der Position L der Hydraulikpumpe 58 ab, die üblicherweise über einen Wegaufnehmer 56 gemessen wird. Darüber hinaus wird als weitere Kompensationsgröße der Gegendruck P_{FR} berücksichtigt, der durch den Flusswiderstand im System, d. h. in der Pumpe 50 und in der als Disposable ausgestalteten Pumpenkammer 53 entsteht. Dieser zu berücksichtigende Gegendruck ist abhängig von der Geschwindigkeit v im System.

Schließlich ist der hydrostatische Druck P_{Stat} zu berücksichtigen, der sich aufgrund der Lage des Patienten ergibt.

Üblicherweise wird wie folgt vorgegangen:
Die Membrankompensation wird initial eingemessen. Dabei verfährt die Hydraulikpumpe 58 über den gesamten Arbeitsweg und nimmt in äquidistanten Abständen, die über die Wegaufnehmer 56 verifiziert werden, den anstehenden Druckwert auf und legt ihn in einer Kurve ab. Diese Kurve ermöglicht es, den ursprünglich gemessenen Rohwert des Hydraulikdrucks P_{Hyd} um den Gegendruck der Membran P_{Memb-ran} zu kompensieren. Die Geschwindigkeitskompensation, d. h. der Gegendruck P_{FR}, der durch den Volumenstromwiderstand hervorgerufen wird, ist fest im Verfahren abgelegt und muß daher nicht eingemessen werden. Dieser wird für die Konfiguration vorab bestimmt und in einem entsprechenden Speicher abgelegt.
Die Erfassung des hydrostatischen Patientendrucks P_{Stat} ist zu Beginn einer jeden Auslaufphase möglich. Beim Befüllen des Dialysats bzw. beim Auslassen des Dialysats wird dem System üblicherweise ein Sollvolumenstrom für die Phasen "Fill/Drain" vorgegeben. Ziel ist es dabei, einen kontinuierlichen Fluss herzustellen. Das Erreichen des Ziels wird durch die Verwendung von zwei diskontinuierlichen Pumpen erschwert.

Wie in dem Geschwindigkeitswegdiagramm in Figur 15 gezeigt, wird eine einzelne Pumpkammer am Anfang des Pumphubes beschleunigt und am Ende des Pumphubes abgebremst. Während der Beschleunigungsphase, d. h. dem sogenannten Anrampen und der Abbremsphase, d. h. dem sogenannten Abrampen variiert der gepumpte Dialysatvolumenstrom. Um dies zu vermeiden, wird wie in Figur 15 schematisch dargestellt, die Abrampung einer ersten Pumpkammer mit der Anrampung der zweiten Pumpkammer derart überlagert, daß sich ein konstanter Dialysatvolumenstrom ergibt.

Gemäß dem Stand der Technik wird der vorgegebene Volumenstrom des Dialysats an der zuvor beschriebenen Pumpe mittels des Wegaufnehmers 56 eingeregelt. Dies führt allerdings zur Druckveränderung im Gesamtsystem, die bewertet werden muß. Bei der Überschreitung eines Grenzwertes P_{PatMax} wird die Bewegung der Pumpe gestoppt. Dieser Grenzwert entspricht der Überschreitung einer maximal zugelassenen Patientendruckgrenze. Um einen kontinuierlichen Volumenstrom bei Betrieb der beiden Pumpen zu erlangen, wird, wie zuvor ausgeführt, ein Anrampen des Volumenstroms in den Kammerenden vorgenommen (vgl. Figur 15). Aufgrund dieser Ansteuerung ergeben sich im System folgende Voraussetzungen und Eigenschaften:
Zunächst muß die Membranspannung P_{Membran} zu Beginn einmalig über den ganzen Bereich als Kurve abhängig von der Pumpenposition aufgenommen werden.

Das bedeutet, daß die Ermittlung des Patientendrucks durch diese Kompensation geprägt ist, was zu Ungenauigkeiten führt, da das zu berücksichtigende Längensignal des Längensensors nur vergleichsweise schwer zu ermitteln ist.

Da die Geschwindigkeit kontinuierlich eingeregelt wird, besitzt das System die Eigenschaft, auf Verschlüsse der Patientenzuleitung oder bei einem "entleerten" Patienten (gegen Ende eines Zyklus) die eingesetzte Energie im Regler zu erhöhen. Dies ruft unweigerlich die Überschreitung des vorab angegebenen Grenzdrucks P_{PatMax} hervor.

Schließlich erhöht sich die Eigenspannung der Membran an den Kammerenden sehr stark und wirkt gegen die Geschwindigkeitsregelung, wodurch die Überlagerung beim Anlaufen bzw. Auslaufen der Pumpsysteme erschwert wird.

Nachteilig bei dem vorbekannten System zur Einstellung des kontinuierlichen Dialysatvolumenstroms sind insbesondere die hohen Ansprüche, die an die Regelstrecke bzw. das Messsystem zu stellen sind. Da diese Anforderungen häufig nicht in gewünschtem Maß erreicht werden, kann es zu einem ungleichmäßigen Lauf der Hydraulikpumpen kommen.

Aufgabe der vorliegenden Erfindung ist es daher, ein Verfahren zur Einstellung eines kontinuierlichen Dialysatvolumenstroms an die Hand zu geben, das mit einfachen Mitteln, d. h. geringeren Anforderungen an Regelsysteme und Messeinrichtungen zu einer kontinuierlichen Förderung des Dialysats führt.

Die vorgenannte Aufgabe wird erfindungsgemäß durch die Kombination der Merkmale des Anspruchs 1 gelöst.

Demnach ist eine Dialysemaschine mit mindestens zwei diskontinuierlichen Pumpen und einer Steuerung zur Erzeugung eines kontinuierlichen Sollvolumenstroms des Dialysats dadurch gekennzeichnet, daß die Energie zum Antrieb der Pumpe mit einem entsprechend dem Pump-Zeit-Volumen der jeweiligen Pumpe und dem geförderten Volumen bestimmten Wert konstant eingestellt wird. Erfindungsgemäß wird hier eine konstante Energie für einen Pumpenhub bestimmt. Dabei wird zur Bestimmung der Energie eine konstante Leistung eingestellt. Diese führt zu einer Volumenstrom-Druckänderung im Gesamtsystem, die bewertet werden muß. Da das Pump-Zeit-Volumen und die geförderte Menge an Dialysat bekannt sind, kann die konstante Energie für den nächsten Pumpenhub bestimmt werden, so daß die Pumpe entsprechend dieser exakt bestimmten konstanten Energie mit exakt dieser Energiemenge betrieben wird. Durch diese Art der Ansteuerung ergeben sich folgende vorteilhafte Eigenschaften des Gesamtsystems:
Bei konstanter Energieeinstellung besitzt das System die Eigenschaft, auf Verschlüsse der Patientenzuleitung oder einen "entleerten" Patienten (gegen Ende eines Zyklus) zu reagieren. Die Last wird größer, so daß bei konstanter Energiemenge die Geschwindigkeit abnimmt.

Zur Bestimmung der Energie kann in einfacher Art und Weise eine konstante elektrische Leistung eingestellt werden.

Aus den sich an den Hauptanspruch anschließenden Unteransprüchen ergeben sich vorteilhafte Ausführungsformen der Erfindung.

So wird der Druck im geförderten Dialysat vorteilhaft gemessen. Bei Überschreiten eines einstellbaren Druckgrenzwertes werden die Pumpen angehalten. Hierdurch kann bei konstant eingestellter Energie das System die Eigenschaft besitzen, auf Verschlüsse der Patientenzuleitung oder einen "entleerten" Patienten (gegen Ende eines Zyklus) zu reagieren, wenn die Energie zu groß eingestellt ist. Die Last wird größer, während die Geschwindigkeit nahezu konstant bleibt, so daß der erzeugte Druck den vorgegebenen Druckgrenzwert übersteigt. In diesem Fall wird die Pumpe angehalten, so daß keine Gefahr besteht, daß der Patient einem zu hohen Dialysatdruck ausgesetzt wird.

Vorteilhaft wird die zur Versorgung der Pumpen eingestellte Energie für jeden weiteren Pumpenhub in einem Pumpenzyklus unter Berücksichtigung des Pump-Zeit-Volumens, des geförderten Volumens sowie gegebenenfalls der Überschreitung des Druckgrenzwertes festgelegt. Hierdurch ergibt sich eine Adaptierung des Systems an gegebenenfalls Laufzeit bedingte Systemänderungen.

Gemäß einer weiteren vorteilhaften Ausgestaltung der Erfindung wird die zur Versorgung der Pumpen eingestellte Energie für den ersten Pumpenhub als Schätzwert aus einer Schätzwerttabelle entnommen. Hier werden entsprechende Erfahrungswerte für das System als Startwert abgelegt.

Soweit als diskontinuierliche Pumpen Membranpumpen zum Einsatz kommen, wird gemäß einer weiteren vorteilhaften Ausgestaltung der Erfindung die Membrancharakteristik berücksichtigt. Dabei kann die Membranspannung für den Fall bestimmt werden, in dem die Membranpumpe am Ende eines Pumphubes nicht mehr pumpt (sogenanntes Abrampen). Des Weiteren kann vorteilhaft der Punkt der Membranspannung bestimmt werden, an dem keine Membranspannung mehr wirkt. Diese beiden Membranwerte reichen für die Durchführung des erfindungsgemäßen Verfahrens aus. Es ist im Unterschied zum Stand der Technik nicht mehr notwendig, über den gesamten Pumpenhub die Membrancharakteristik aufzunehmen und in einem entsprechenden Speicher abzulegen. Dadurch wird das Verfahren weiter vereinfacht.

Bei der Dialysemaschine weist die Steuereinheit erfindungsgemäß Mittel zur Bestimmung der für einen Pumpenzyklus der Pumpen einzusetzenden Energie auf. Hier kann die Energie über eine Strom- oder Spannungsmessung bestimmt werden.

Gemäß einer vorteilhaften Ausgestaltung der Dialysemaschine bestehen die diskontinuierlichen Pumpen aus Membranpumpen. Weiterhin sind vorzugsweise Mittel zur Bestimmung des Membrandrucks der Pumpenmembranen vorgesehen.

Weitere Merkmale, Einzelheiten und Vorteile der Dialysemaschine, in der das erfindungsgemäße Verfahren eingesetzt wird, ergeben sich aus der nachfolgenden ausführlichen Erläuterung der Dialysemaschine. Dabei zeigen:
- Fig. 1: drei Diagramme, welche typische Verläufe einer automatischen Peritonealdialysebehandlung zeigen,
- Fig. 2: eine Prinzipdarstellung eines Peritonealdialysesystems,
- Fig. 3: eine Prinzipdarstellung der Aufteilung des Peritonealdialysesystems in eine Dialysemaschine und ein Fluidsystem,
- Fig. 4: ein erstes Ausführungsbeispiel einer Kassette,
- Fig. 5: ein zweites Ausführungsbeispiel einer Kassette,
- Fig. 6: eine perspektivische Ansicht eines ersten Ausführungsbeispiels einer Dialysemaschine,
- Fig. 7: ein Flussdiagramm eines ersten Ausführungsbeispiels eines Peritonealdialysesystems,
- Fig. 8: eine perspektivische Ansicht eines zweiten Ausführungsbeispiels einer Dialysemaschine,

- Fig. 9: ein Flussdiagramm eines zweiten Ausführungsbeispiels eines Peritonealdialysesystems,
- Fig. 10: das Ankoppeln der Kassette beim zweiten Ausführungsbeispiels eines Peritonealdialysesystems,
- Fig. 11: ein erstes Ausführungsbeispiels eines Pumpaktors,
- Fig. 12: das Ankoppeln eines Pumpbereichs der Kassette an einen Pumpaktor,
- Fig. 13: eine Prinzipdarstellung des Aufbaus eines Ausführungsbeispiels einer Steuerung,
- Figur 14: eine Prinzipdarstellung des Pumpsystems eines Peritonealdialysesystems,
- Figur 15: ein Geschwindigkeits-Weg Diagramm zur Verdeutlichung der Kinematik der Pumpkammern und
- Figur 16: ein Diagramm zur Darstellung des Druckverlaufs in einer Pumpkammer.

Im folgenden soll die Funktion einer Dialysemaschine, bei welcher die vorliegende Erfindung zum Einsatz kommt, zunächst allgemein beschrieben werden. Bei der Dialysemaschine handelt es sich dabei im Ausführungsbeispiel um eine Peritonealdialysemaschine. Die unten beschriebenen Komponenten können jedoch in gleicher oder ähnlicher Weise auch für eine Hämodialysemaschine eingesetzt werden.

Die Peritonealdialyse ist eine Variante der künstlichen Blutwäsche, bei welcher das gut durchblutete Bauchfell (Peritoneum) des Patienten als körpereigene Filtermembran verwendet wird. Hierfür wird über einen Katheter Dialysat in die Bauchhöhle eingeführt. Nach dem Prinzip der Osmose diffundieren nun Harnbestandteile des Blutes durch das Bauchfell in das in der Bauchhöhle befindliche Dialysat. Nach einer gewissen Verweilzeit wird das Dialysat mit den Harnbestandteilen wieder aus der Bauchhöhle ausgelassen.

Bei der automatischen Peritonealdialyse steuert und überwacht eine Dialysemaschine die Einführung des frischen Dialysats in die Bauchhöhle und das Auslassen des verbrauchten Dialysats. Eine solche Dialysemaschine, auch Cycler genannt, befüllt und entleert die Bauchhöhle dabei üblicherweise mehrmals über Nacht, d.h. während der Patient schläft.

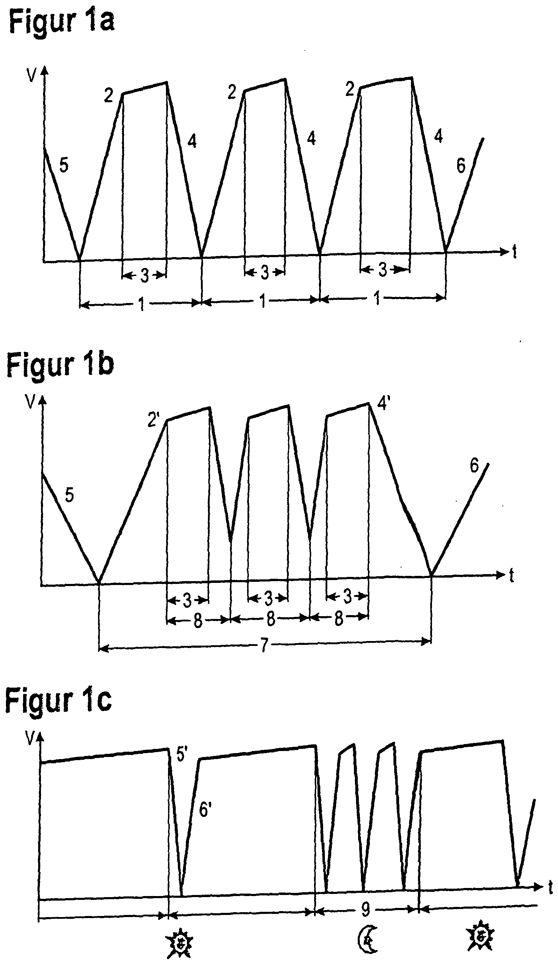

In Figuren 1a bis 1c sind drei unterschiedliche Verfahrensabläufe gezeigt, wie sie von einer Dialysemaschine durchgeführt werden. Einer oder mehrerer dieser Verfahrensabläufe ist dabei üblicherweise in der Steuerung der Dialysemaschine hinterlegt. Dabei ist es üblicherweise möglich, die abgespeicherten Verfahrensabläufe an den Patienten anzupassen.

Bei den Figuren 1a bis 1c ist jeweils die in der Bauchhöhle des Patienten befindliche Dialysatmenge V über die Zeit t aufgezeichnet. Figur 1a zeigt dabei den Verlauf einer normalen automatischen Peritonealdialysebehandlung über Nacht. Zu Beginn der Behandlung erfolgt dabei zunächst ein initialer Auslauf 5, durch welchen Dialysat, das über den Tag in der Bauchhöhle des Patienten belassen worden war, entnommen wird. Daraufhin folgen mehrere Behandlungszyklen 1, in Figur 1a drei aufeinander folgende Behandlungszyklen 1. Jeder Behandlungszyklus besteht dabei aus einer Einlaufphase 2, einer Verweilphase 3 und einer Auslaufphase 4. Während der Einlaufphase 2 wird dabei ein gewisses Volumen an frischer Dialyseflüssigkeit in die Bauchhöhle des Patienten eingelassen. Die maximal zulässige Dialysatmenge beträgt dabei je nach Patienten zwischen ca. 1,5 und 3 I. Das frische Dialysat verbleibt nun über eine gewisse Verweilzeit 3 in der Bauchhöhle. Typischerweise dauert die Verweilphase dabei einige Stunden. Daraufhin wird das nun verbrauchte Dialysat in der Auslaufphase 4 wieder aus der Bauchhöhle herausgelassen. Daraufhin startet ein neuer Behandlungszyklus. Die Behandlung wird mit einem letzten Einlauf 6 abgeschlossen, durch welchen eine gewisse Menge an frischem Dialysat in die Bauchhöhle des Patienten eingeführt wird. Diese verbleibt dann über den Tag in der Bauchhöhle des Patienten.

Die einzelnen Behandlungszyklen 1, welche über Nacht erfolgen, werden dabei automatisch von der Steuerung der Dialysemaschine angesteuert. Der initiale Auslauf und der letzte Einlauf können ebenfalls automatisch von der Dialysemaschine angesteuert werden. Alternativ werden diese manuell von einer Bedienperson oder vom Patienten aktiviert.

In Figur 1b ist eine sogenannte Tidalbehandlung gezeigt. Auch diese beginnt mit einem initialen Auslauf 5 und endet mit einem letzten Einlauf 6. Weiterhin ist ein Basiszyklus 7 vorgesehen, welcher sich in mehrere Tidalzyklen 8 aufteilt. Dabei ist zunächst eine Basiseinlaufphase 2' vorgesehen. Nach der Verweilphase 3 wird jedoch nicht mehr das komplette Dialysatvolumen aus der Bauchhöhle entnommen, sondern nur eine gewisse Teilmenge des in der Bauchhöhle befindlichen Dialysats. Dieses wird dann durch ein entsprechendes Volumen an frischem Dialysat ersetzt. Nach einem erneuten Verweilzyklus kann eine weitere Tidalentnahme erfolgen, bei welcher nicht das gesamte im Bauchraum befindliche Dialysat entfernt wird. Zum Ende des Basiszyklus 7 erfolgt eine Basisauslaufphase 4', bei welcher nun das gesamte Dialysat entnommen wird. In Figur 1b ist dabei lediglich ein Basiszyklus 1 dargestellt. Alternativ können jedoch auch mehrere Basiszyklen vorgesehen sein.

In Figur 1c ist der Verlauf einer Peritonealdialysebehandlung mit einer sogenannten PD-Plus-Behandlung gezeigt. Dabei erfolgt während der Nacht 9 eine übliche Peritonealdialysebehandlung, welche z. B. gemäß den Figuren 1a oder 1b durchgeführt werden kann. Weiterhin ist jedoch während des Tages eine zusätzliche PD-Plus-Behandlung vorgesehen, bei welcher in einer Auslaufphase 5' das verbrauchte Dialysat entnommen und in einer Einlaufphase 6' durch frisches Dialysat ersetzt wird. Bei der PD-Plus-Behandlung wird damit eine normale nächtliche Peritonealdialysebehandlung mit einem oder mehreren zusätzlichen Behandlungszyklen während des Tages kombiniert. Der Verlauf der nächtlichen Behandlung wird dabei wie üblich automatisch durch die Dialysemaschine durchgeführt. Die Behandlungszyklen während des Tages werden ebenfalls über die Maschine durchgeführt und überwacht.

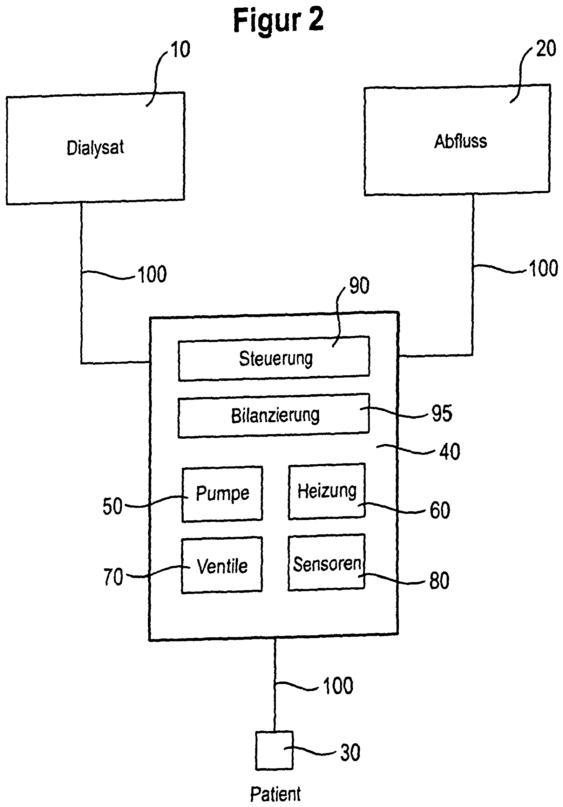

In Figur 2 ist nun der Aufbau eines typischen Peritonealdialysesystems schematisch dargestellt. Das Peritonealdialysesystem umfaßt dabei einen Behälter 10 mit frischem Dialysat und einen Abfluss 20 für gebrauchtes Dialysat. Weiterhin ist ein Konnektor 30 vorgesehen, welcher an einen Katheter des Patienten angeschlossen werden kann, um entweder frisches Dialysat in die Bauchhöhle des Patienten einzuführen oder gebrauchtes Dialysat aus der Bauchhöhle abzuführen. Der Behälter 10 mit frischem Dialysat, der Abfluss 20 für gebrauchtes Dialysat und der Konnektor 30 zum Patienten sind dabei über Fluidwege 100 miteinander verbunden und bilden zusammen mit diesen das Fluidsystem des Peritonealdialysesystems.

Zur Durchführung der Peritonealdialysebehandlung ist eine Dialysemaschine 40, auch Cycler genannt, vorgesehen. Die Dialysemaschine 40 umfaßt dabei folgende Hauptkomponenten:
- Eine Pumpe 50, welche zum Transport der Flüssigkeiten eingesetzt wird. Die Pumpe 50 befördert dabei das frische Dialysat vom Behälter 10 zum Konnektor 30. Weiterhin kann die Pumpe 50 das verbrauchte Dialysat vom Konnektor 30 zum Abfluss 20 transportieren.
- Ventile 70, welche zur Steuerung der Flüssigkeitsströme eingesetzt werden. Die Ventile 70 öffnen und schließen die Fluidwege 100, um so die entsprechenden Fluidverbindungen zwischen dem Behälter 10, dem Konnektor 30 und dem Abfluss 20 herzustellen.
- Eine Heizung 60, welche das frische Dialysat auf eine Temperatur von ca. 37°C bringt, bevor dieses dem Patienten zugeführt wird. Da bei der Peritonealdialyse relativ große Mengen an Dialysat direkt in die Bauchhöhle des Patienten eingeführt werden, ist die Heizung 60 nötig, um den Patienten nicht zu unterkühlen und um ein unangenehmes Gefühl durch zu kaltes Dialysat zu vermeiden.
- Sensoren 80, über welche der ordnungsgemäße Ablauf der Behandlung überwacht und/oder gesteuert werden kann. Insbesondere können dabei Temperatursensoren zum Einsatz kommen. Weiterhin können gegebenenfalls Drucksensoren zum Einsatz kommen.

Alle Komponenten der Dialysemaschine 40 werden dabei über eine Steuerung 90 angesteuert. Die Steuerung 90 steuert dabei insbesondere die Pumpe 50, die Heizung 60 und die Ventile 70 auf Grundlage der Daten der Sensoren 80 an. Die Steuerung 90 sorgt dabei für den automatischen Ablauf der Peritonealdialyse. Als wichtige Komponente umfaßt die Steuerung 90 dabei eine Bilanzierung 95, welche die dem Patienten zugegebenen und entnommenen Flüssigkeitsmengen bilanziert. Die Bilanzierung verhindert dabei, daß dem Patienten zuviel Flüssigkeit zugegeben oder zuviel Flüssigkeit entnommen wird.

Die Bilanzierung 95 kann dabei allein auf Grundlage der Ansteuerdaten und/oder der Sensordaten für die Pumpe 50 erfolgen. Alternativ kann die Bilanzierung auch über separat vorgesehene Bilanzierkammern erfolgen. Ebenso ist es möglich, zur Bilanzierung eine Waage einzusetzen. Eine solche Waage wiegt beispielsweise das Gewicht des Behälters 10 mit frischem Dialysat und/oder eines Behälters 20 mit gebrauchtem Dialysat.

Da bei der Peritonealdialyse das Dialysat dem Patienten direkt in die Bauchhöhle verabreicht wird, ist auf äußerste Sterilität zu achten. Daher werden die Fluidwege bzw. das Fluidsystem, welches mit dem frischen und/oder dem gebrauchten Dialysat in Kontakt kommt, üblicherweise als Einwegteil ausgeführt. Insbesondere werden die Fluidwege bzw. das Fluidsystem dabei als Kunststoffteile ausgeführt. Diese können so in einer sterilen Umverpackung angeliefert und erst kurz vor der Behandlung ausgepackt werden.

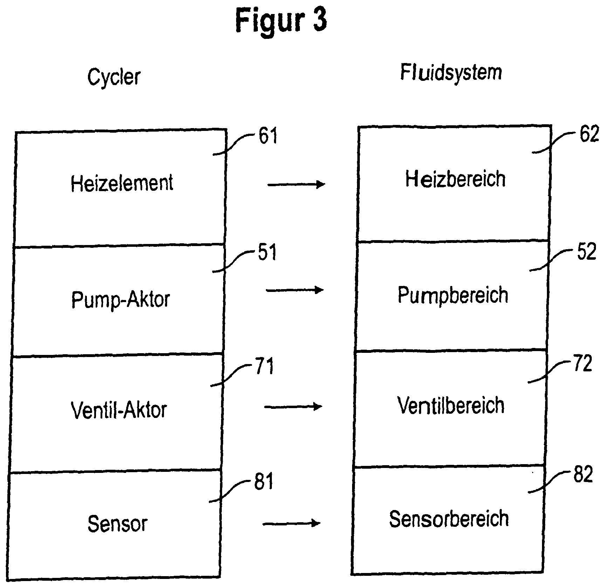

Um dennoch eine Steuerung der Peritonealdialyse durch die Dialysemaschine 40 zu ermöglichen, muß das Fluidsystem an die Dialysemaschine 40 angekoppelt werden. In Figur 3 ist dabei schematisch dargestellt, wie einzelne Elemente der Dialysemaschine 40 an entsprechende Bereiche des Fluidsystems angekoppelt werden.

Die Dialysemaschine 40 weist dabei ein Heizelement 61 auf. Dieses muß an einen entsprechenden Heizbereich 62 des Fluidsytems angekoppelt werden. Die Ankopplung ermöglicht dabei die Übertragung von Wärmeenergie von dem Heizelement 61 an das im Heizbereich 62 befindliche Dialysat.

Die Dialysemaschine 40 weist weiterhin einen oder mehrere Pumpaktoren 51 auf, welche mit einem Pumpbereich 52 des Fluidsystems gekoppelt werden. Die Pumpaktoren 51 erzeugen dabei eine Pumpkraft, welche auf den Pumpbereich 52 übertragen wird. Hierdurch kann die im Pumpbereich 52 befindliche Flüssigkeit entlang der Fluidwege bewegt werden.

Weiterhin weist die Dialysemaschine einen oder mehrere Ventilaktoren 71 auf. Diese erzeugen eine Schließbewegung, welche auf entsprechende Ventilbereiche 72 der Fluidwege übertragen wird. Hierdurch können die Ventilbereiche 72 der Fluidwege entsprechend geschlossen bzw. geöffnet werden.

Weiterhin weist die Dialysemaschine einen oder mehrere Sensoren 81 auf. Diese werden an einen entsprechenden Sensorbereiche 82 des Fluidsystems angekoppelt. Hierdurch können die Sensoren 81 gewisse Eigenschaften des Dialysats messen. Insbesondere kann hierdurch die Temperatur des Dialysats gemessen werden. Weiterhin kann vorgesehen sein, daß der Druck in dem Fluidsystem bestimmt wird.

Selbstverständlich weist die Dialysemaschine gegebenenfalls noch weitere Aktoren und/oder Sensoren auf, welche nicht mit den Fluidwegen gekoppelt werden müssen.

Die einzelnen Komponenten eines Peritonealdialysesystems sollen nun im folgenden näher anhand von Ausführungsbeispielen dargestellt werden.

### 1. Fluidsystem

### 1.1 Dialysatbehälter

Frisches Dialysat wird üblicherweise in Kunststoffbeuteln zur Verfügung gestellt. Solche Kunststoffbeutel weisen üblicherweise zwei Lagen von Kunststoffolie auf, welche in einem Randbereich miteinander verschweißt sind und so einen Behälter bilden, welcher mit frischem Dialysat befüllt ist. An diesen Behälter ist üblicherweise ein Schlauchelement angeschweißt, durch welches das Dialysat aus dem Beutel entnommen werden kann. An dem Schlauchelement ist üblicherweise ein Konnektor angeordnet, über welchen der Dialysatbehälter mit den übrigen Fluidwegen verbunden werden kann. Weiterhin weist der Beutel üblicherweise an der dem Schlauch gegenüberliegenden Seite eine Aussparung oder Öse auf, über welche der Beutel an einen Haken aufgehängt werden kann. Hierdurch kann sichergestellt werden, daß das Dialysat problemlos aus dem Beutel abfließt.

Das Dialysat besteht üblicherweise aus einem Puffer, einem Osmotikum und Elektrolyten. Als Puffer kann dabei z. B. Bicarbonat eingesetzt werden. Als Osmotikum wird üblicherweise Glucose eingesetzt. Alternativ können auch Glucosepolymere oder Glucosepolymerderivate eingesetzt werden. Die Elektrolyte umfassen üblicherweise Kalzium und Natrium.

Das Dialysat kann dabei hitzesterilisiert werden. Dies erfolgt vorteilhafterweise, nachdem das Dialysat in den Beutel gefüllt wurde. Hierdurch werden sowohl das Dialysat als auch der Beutel hitzesterilisiert. Dabei wird der gefüllte Beutel üblicherweise zunächst in eine Umverpackung verpackt, woraufhin das gesamte System sterilisiert wird.

Da die fertige Dialysatlösung je nach Inhaltsstoffen oft nicht hitzesterilisiert werden kann oder nicht lange gelagert werden kann, kann vorgesehen sein, einzelne Komponenten des Dialysats getrennt zu lagern und erst kurz vor der Behandlung zusammenzuführen. Eine erste Einzellösung umfaßt dabei üblicherweise den Puffer, während eine zweite Einzellösung Glucose und Elektrolyte umfaßt. Gegebenenfalls können auch mehr als zwei Einzellösungen und damit mehr als zwei Bereiche in einem Beutel vorgesehen sein. Dabei kann ein Mehrfachkammerbeutel, insbesondere ein Doppelkammerbeutel vorgesehen sein, welcher mehrere getrennte Bereiche zur Lagerung der Einzellösungen aufweist. Diese Bereiche sind durch ein Verbindungselement getrennt, welches mechanisch geöffnet werden kann, um die Einzelflüssigkeiten miteinander zu vermischen. Insbesondere kann dabei eine sogenannte Peel-Naht zwischen den beiden Bereichen des Beutels vorgesehen sein, welche sich bei Anwendung eines bestimmten Druckes auf mindestens einen der Bereiche des Beutels öffnet.

Da während einer nächtlichen Peritonealdialysebehandlung relativ große Mengen an Dialysat verbraucht werden, werden üblicherweise mehrere Dialysatbehälter parallel eingesetzt. Diese werden über entsprechende Konnektoren mit den Fluidwegen verbunden und können durch eine entsprechende Schaltung der Ventilen zum Befüllen des Patienten herangezogen werden.

### 1.2 Abfluss

Zum Entsorgen der verbrauchten Dialysierflüssigkeit kann diese entweder sofort in die Kanalisation abgeführt oder zunächst in einem Abflussbehälter gesammelt werden. Als Abflussbehälter wird dabei üblicherweise ebenfalls ein Beutel eingesetzt. Dieser ist vor Beginn der Behandlung leer und kann so das verbrauchte Dialysat aufnehmen. Der Beutel kann dann nach Beendigung der Behandlung entsprechend entsorgt werden.

### 1.3 Kassette

Wie bereits eingangs beschrieben, weist das Fluidsystem eine Mehrzahl von Bereichen auf, in welchen die Dialysemaschine auf das Fluidsystem einwirken muß. Hierfür muß das Fluidsystem an die Dialysemaschine angekoppelt werden.

Um das Ankoppeln der Fluidwege an die Dialysemaschine und die Einwirkung der entsprechenden Elemente der Dialysemaschine auf die Fluidwege zu vereinfachen, werden Kassetten eingesetzt. In einer solchen Kassette sind mehrere Bereiche, in welchen die Dialysemaschine auf die Fluidwege einwirkt, gemeinsam angeordnet. Hierfür weist eine Kassette üblicherweise ein Hartteil aus Kunststoff auf, in welches zu einer Seite hin offene Kammern als Fluidwege eingebracht sind. Diese Kammern werden von einer flexiblen Kunststoffolie bedeckt, welche für die Ankopplung an die Dialysemaschine sorgt. Die flexible Kunststoffolie ist dabei üblicherweise in einem Randbereich mit dem Hartteil verschweißt. Die Kassette wird mit einer Ankoppelfläche der Dialysemaschine verpreßt, so daß die Aktoren und/oder Sensoren der Dialysemaschine mit entsprechenden Bereichen der Kassette in Kontakt kommen.

Die Kassette weist weiterhin Anschlüsse zum Anschluss des Dialysatbehälters 10, des Konnektors 30 sowie des Ablaufs 20 auf.

Eine Kassette umfaßt dabei üblicherweise zumindest einen Pumpbereich und einen oder mehrere Ventilbereiche. Über die Kassette kann so der Flüssigkeitstransport durch das Fluidsystem gesteuert werden. Weiterhin kann die Kassette Sensorbereiche aufweisen, welche eine einfache Ankopplung von Sensoren der Dialysemaschine an das Fluidsystem ermöglichen. Gegebenenfalls kann die Kassette weiterhin einen oder mehrere Heizbereiche aufweisen, welche an entsprechende Heizelemente der Dialysemaschine ankoppelbar sind.

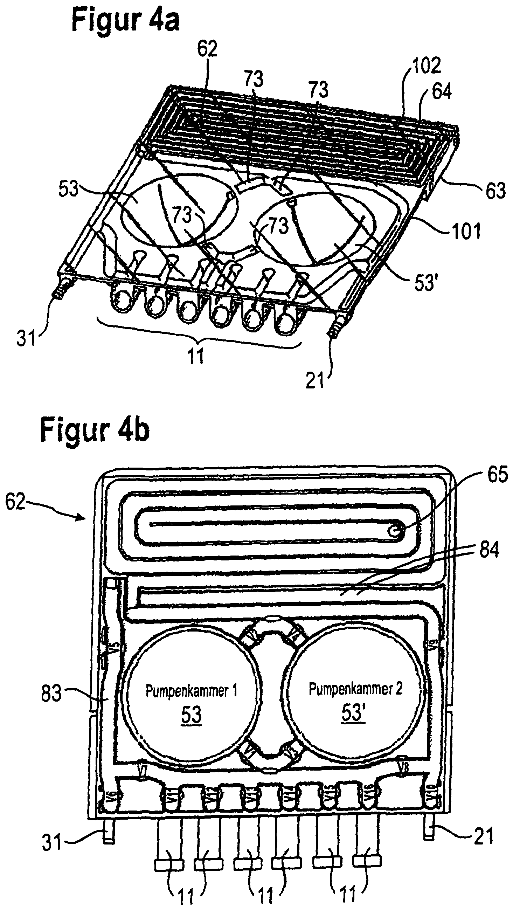

In Figuren 4a und 4b ist ein erstes Ausführungsbeispiel einer Kassette dargestellt. Diese weist ein Hartteil 101 aus Kunststoff auf, in welchem die Fluidwege und Ankopplungsbereiche als entsprechende Aussparungen, Kammern und Kanäle eingebracht sind. Das Hartteil kann dabei z. B. als Spritzgussteil oder als Tiefziehteil gefertigt werden. Die Ankoppelebene des Hartteils 101 wird von einer flexiblen Folie 102 bedeckt, welche in einem Randbereich mit dem Hartteil verschweißt ist. Durch das Verpressen der Kassette mit einer Ankoppelfläche der Dialysemaschine wird die flexible Folie 102 mit dem Hartteil verpreßt. Durch das Verpressen der flexiblen Folie mit den Stegbereichen des Hartteils werden die Fluidwege innerhalb der Kassette fluiddicht voneinander getrennt.

Die Kassette weist Anschlüsse zum Anschluss der Kassette an die übrigen Fluidwege auf. Zum einen ist ein Anschluss 21 zum Anschluss an den Abfluss 20 sowie ein Anschluss 31 zum Anschluss an den Konnektor 30 vorgesehen. An diesen Anschlüssen können entsprechende Schlauchelemente vorgesehen sein, welche in Figur 4a nicht dargestellt sind. Weiterhin weist die Kassette eine Mehrzahl von Anschlüssen 11 zum Anschluss von Dialysatbehältern 10 auf. Die Anschlüsse 11 sind dabei im ersten Ausführungsbeispiel als Konnektoren ausgeführt, an welche entsprechende Konnektorelemente angeschlossen werden können.

Die Anschlüsse stehen jeweils mit Fluidwegen innerhalb der Kassette in Verbindung. In diesen Fluidwegen sind Ventilbereiche vorgesehen. In diesen Ventilbereichen kann die flexible Folie 102 über maschinenseitige Ventilaktoren so in das Hartteil 101 gedrückt werden, daß der entsprechende Fluidweg versperrt ist. Die Kassette weist dabei zunächst einmal für jeden Anschluss ein entsprechendes Ventil auf, über welches dieser Anschluss geöffnet bzw. geschlossen werden kann. Dem Anschluss 21 für den Abfluss 20 ist dabei das Ventil V10 zugeordnet, dem Anschluss 31 für den Patientenkonnektor 30 das Ventil V6. Den Anschlüssen 11 für die Dialysatbehälter 10 sind die Ventile V11 bis V16 zugeordnet.

Weiterhin sind in der Kassette Pumpenkammern 53 und 53' vorgesehen, welche durch entsprechende Pumpaktoren der Dialysemaschine betätigt werden können. Bei den Pumpenkammern 53 und 53' handelt es sich dabei um konkave Aussparungen in dem Hartteil 101, welche von der flexiblen Folie 102 bedeckt werden. Durch Pumpaktoren der Dialysemaschine kann die Folie nun in die Pumpenkammern 53 und 53' hineingedrückt bzw. wieder aus diesen Pumpenkammern herausgezogen werden. Hierdurch kann, im Zusammenspiel mit den Ventilen V1 bis V4, welche die Zugänge und Abläufe der Pumpenkammern 53 und 53' schalten und in Fig. 4a mit dem Bezugszeichen 73 bezeichnet wurden, ein Pumpstrom durch die Kassette erzeugt werden. Die Pumpkammern sind dabei über entsprechende Ventilschaltungen mit allen Anschlüssen der Kassette verbindbar.

Weiterhin ist ein Heizbereich 62 in die Kassette integriert. In diesem Bereich wird die Kassette mit Heizelementen der Dialysemaschine in Kontakt gebracht, welche das durch diesen Bereich der Kassette fließende Dialysat erwärmen. Der Heizbereich 62 weist dabei einen Kanal für das Dialysat auf, welcher sich spiralförmig über den Heizbereich 62 erstreckt. Der Kanal wird dabei durch Stege 64 des Hartteils gebildet, welche von der flexiblen Folie 102 abgedeckt sind.

Der Heizbereich 62 ist dabei auf beiden Seiten der Kassette vorgesehen. Hierfür ist auch auf der Unterseite 63 der Kassette im Heizbereich eine flexible Folie am Hartteil angeordnet. Die flexible Folie ist dabei ebenfalls in einem Randbereich mit dem Hartteil verschweißt. Auf der Unterseite ist ebenfalls ein Kanal angeordnet, durch welchen das Dialysat fließt. Die Kanäle auf der Unterseite und der Oberseite werden dabei durch eine mittlere Platte des Hartteils gebildet, welche die Oberseite von der Unterseite trennt, und auf welcher nach unten und nach oben Stege vorgesehen sind, welche die Kanalwandungen bilden. Dabei fließt das Dialysat zunächst spiralförmig auf der Oberseite bis zum Durchbruch 65 durch die mittlere Platte, von wo aus das Dialysat auf der Unterseite durch den entsprechenden Kanal zurückfließt. Durch den auf der Ober- und der Unterseite vorgesehenen Heizbereich kann die Heizfläche, welche zum Aufheizen der Flüssigkeit zur Verfügung steht, entsprechend vergrößert werden. Selbstverständlich ist aber auch eine Ausführungsform der Kassette möglich, bei welcher nur auf einer Seite der Kassette ein Heizbereich angeordnet ist.

Weiterhin sind Ausführungsformen der Kassette möglich, bei welcher ein Heizelement in die Kassette integriert ist. Insbesondere kann dabei ein elektrisches Heizelement wie z.B. Heizwendel in das Hartteil der Kassette eingegossen sein. Hierdurch kann auf ein maschinenseitiges Heizelement verzichtet werden und die Durchflussheizung in die Kassette integriert werden. Dabei sind an der Kassette elektrische Kontakte zur Konnektierung des elektrischen Heizelements angeordnet.

Die Kassette weist weiterhin Sensorbereiche 83 und 84 auf, durch welche Temperatursensoren der Dialysemaschine an die Kassette gekoppelt werden können. Die Temperatursensoren liegen dabei auf der flexiblen Folie 102 auf und können so die Temperatur der durch den darunter liegenden Kanal fließenden Flüssigkeit messen. Dabei sind am Eingang des Heizbereiches zwei Temperatursensoren 84 vorgesehen. Am patientenseitigen Ausgang ist ein Temperatursensor 83 vorgesehen, über welchen die Temperatur des zum Patienten gepumpten Dialysats gemessen werden kann.

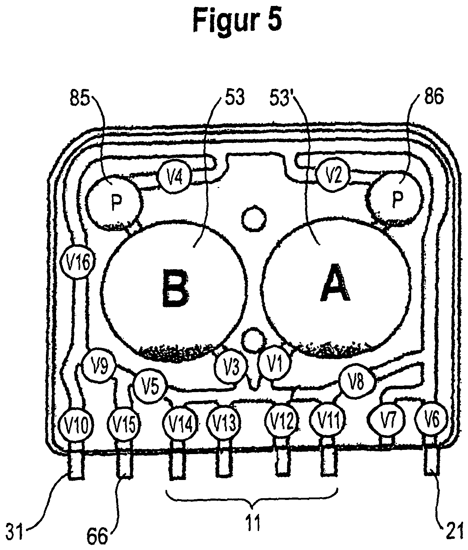

In Figur 5 ist ein zweites Ausführungsbeispiel für eine Kassette gezeigt. Die Kassette entspricht dabei in ihrer Ausführung im wesentlichen dem ersten Ausführungsbeispiel, umfaßt jedoch keinen Heizbereich. Bei Einsatz dieser Kassette erfolgt die Heizung daher nicht wie beim ersten Ausführungsbeispiel gezeigt über einen in die Kassette integrierten Heizbereich, sondern z.B. über einen Heizbeutel, welcher auf eine Heizplatte der Dialysemaschine gelegt wird.

Das in Figur 5 gezeigte zweite Ausführungsbeispiel einer Kassette weist wiederum Fluidwege auf, welche über Ventilbereiche, die hier ebenfalls von V1 bis V16 durchnumeriert sind, geöffnet und geschlossen werden können. Weiterhin weist die Kassette Anschlüsse zum Anschluss an weitere Komponenten des Fluidsystems auf. Dabei ist wiederum der Anschluss 21 zum Anschluss an den Abfluss 20, sowie der Anschluss 31 zum Anschluss an den Konnektor 30 zum Patienten vorgesehen. Weiterhin sind Anschlüsse 11 zum Anschluss von Dialysatbehältern 10 vorgesehen.

Im Unterschied zum ersten Ausführungsbeispiel weist die im zweiten Ausführungsbeispiel gezeigte Kassette einen weiteren Anschluss 66 zum Anschluss eines Heizbeutels auf. Zum Erwärmen der Flüssigkeit aus den Dialysatbehältern 10 kann die Flüssigkeit dabei über den Anschluss 66 in einen Heizbeutel gepumpt werden. Dieser Heizbeutel liegt auf einem Heizelement auf, so daß die im Heizbeutel befindliche Flüssigkeit erwärmt werden kann. Daraufhin wird die Flüssigkeit aus dem Heizbeutel zum Patienten gepumpt.

Die Pumpkammer 53 und 53' und die Ventile V1 bis V4 entsprechen in Aufbau und Funktion den entsprechenden Komponenten im ersten Ausführungsbeispiel.

Im Unterschied zum ersten Ausführungsbeispiel weist die Kassette im zweiten Ausführungsbeispiel keinen Sensorbereich zum Anschluss eines Temperatursensors auf. Dieser ist vielmehr im Bereich der Heizelemente angeordnet. Die Kassette weist jedoch Messbereiche 85 und 86 zum Messen des Drucks in den Pumpekammern 53 und 53' auf. Die Messbereiche 85 und 86 sind dabei Kammern, welche mit dem Pumpkammern fluidisch in Verbindung stehen und ebenfalls von der flexiblen Folie abgedeckt werden. An die Messbereiche können geräteseitige Drucksensoren angekoppelt werden, welche den Druck in den Messkammern 85 und 86 und damit in den Pumpkammern 53 und 53' messen.

Die Verbindung der Anschlüsse 11, 21, 31 und 66 der Kassette mit den weiteren Komponenten des Fluidsystems erfolgt im zweiten Ausführungsbeispiel über Schlauchverbindungen. An diesen Schlauchverbindungen sind gegebenenfalls Konnektoren angeordnet.

### 1.3 Schläuche

Die Verbindung zwischen den einzelnen Behältern des Systems, der Kassette und dem Patientenkonnektor erfolgt üblicherweise über Schlauchverbindungen. Da es sich jeweils um Einwegartikel handelt, sind die Schläuche dabei üblicherweise zumindest auf einer Seite bereits mit einem weiteren Element fest verbunden. Z. B. können Schläuche bereits an einem oder mehreren der Anschlüsse der Kassette vorgesehen sein. Ebenfalls können Schläuche bereits fest mit Beuteln in Verbindung stehen.

### 1.4 Verbindungen

Das Fluidsystem ist üblicherweise in mehrere Teile aufgeteilt und jeweils steril verpackt. Diese Teile müssen für die Behandlung zunächst miteinander verbunden werden. Insbesondere sind dabei üblicherweise die Kassette sowie der oder die Dialysatbeutel getrennt voneinander verpackt.

Die Verbindungen zwischen den einzelnen Elementen des Fluidsystems erfolgt üblicherweise über Konnektoren. Die Konnektoren sind dabei so gestaltet, daß sie eine sterile Verbindung zwischen den einzelnen Komponenten ermöglichen. Dies erfolgt z. B. über entsprechende Schutzfolien, welche beim Schließen des Konnektors automatisch geöffnet werden.

Die Verbindung der einzelnen Komponenten kann dabei manuell durch eine Bedienperson bzw. den Patienten selbst erfolgen. Alternativ kann vorgesehen sein, daß die Verbindung der einzelnen Komponenten durch die Dialysemaschine erfolgt.

Hierzu können z. B. die entsprechenden Konnektoren in eine Konnektoraufnahme der Dialysemaschine eingelegt und automatisch durch die Dialysemaschine zusammengeführt werden.

Weiterhin kann eine elektronische Steuerung vorgesehen sein, welche überwacht, daß die richtigen Komponenten des Systems miteinander verbunden werden. Hierfür können an den Konnektoren Identifikationsmittel wie z. B. Barcodes oder RFIDs vorgesehen sein, welche die Komponenten identifizieren. Die Dialysemaschine umfaßt dabei eine Identifikationsmittel-Erfassungseinheit wie z. B. einen Barcodeleser oder eine RFID-Erfassungseinheit, welcher die Identifikationsmittel auf den Konnektoren erfasst. Hierdurch kann die Steuerung der Peritonealdialyse erkennen, ob die korrekten Konnektoren eingelegt wurden.

Eine solche Überprüfung der korrekten Zusammenstellung des Fluidsystems kann dabei insbesondere mit einer automatischen Konnektion der Konnektoren kombiniert sein. Das System überprüft so zunächst, ob die richtigen Konnektoren in die Konnektorenaufnahmen eingelegt wurden. Die Verbindung zwischen den Konnektoren wird durch die Dialysemaschine nur hergestellt, wenn die richtigen Konnektoren eingelegt wurden. Andernfalls macht die Dialysemaschine den Benutzer darauf aufmerksam, daß die falschen Konnektoren eingelegt wurden.

### 2. Die Dialysemaschine

Die einzelnen Komponenten einer Dialysemaschine sollen nun im folgenden anhand von zwei Ausführungsbeispielen näher beschrieben werden.

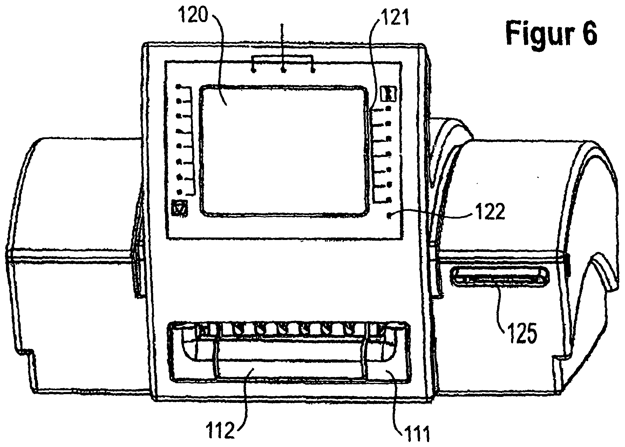

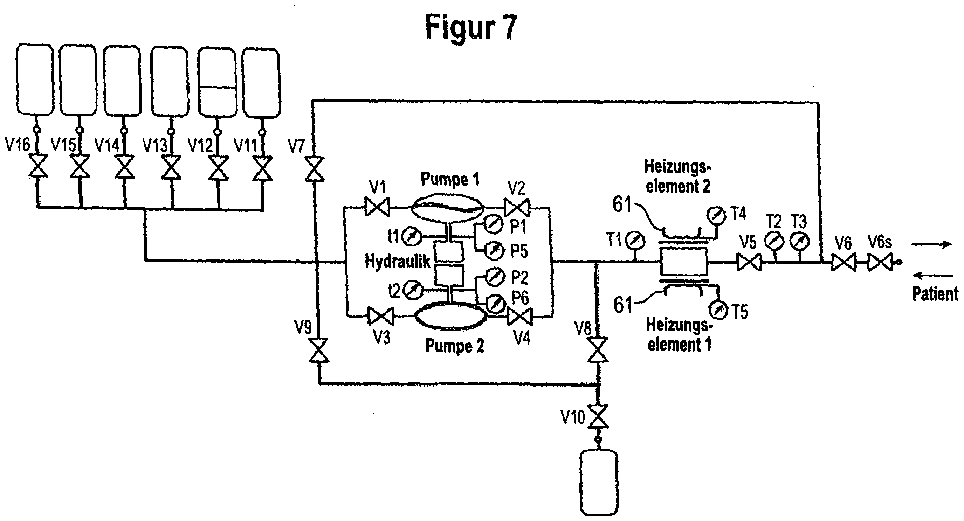

In Figur 6 ist dabei ein erstes Ausführungsbeispiel einer Dialysemaschine gezeigt, bei welchem das erste Ausführungsbeispiel einer Kassette eingesetzt wird. Das sich aus dem ersten Ausführungsbeispiel einer Dialysemaschine und dem ersten Ausführungsbeispiel einer Kassette ergebende Peritonealdialysesystem ist dabei in Figur 7 gezeigt.

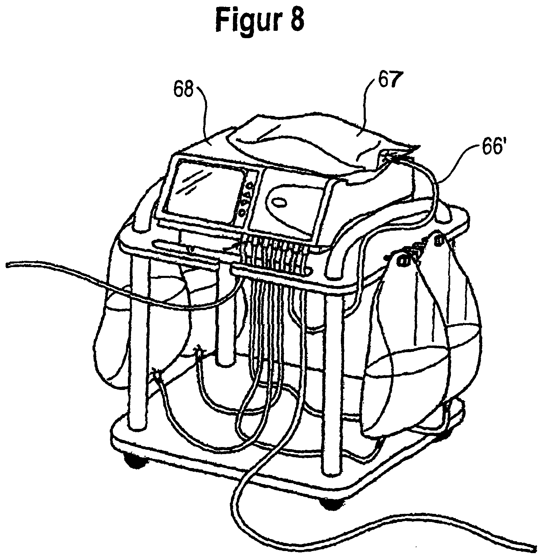

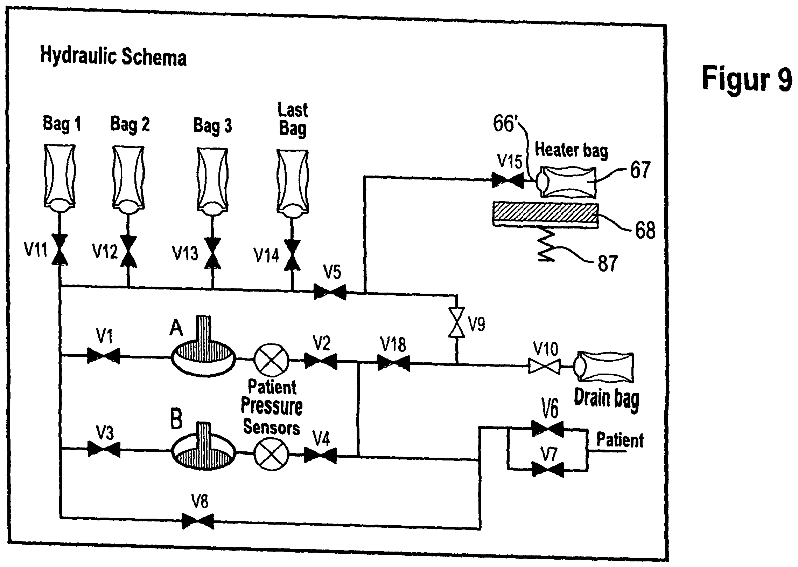

In Figur 8 ist ein zweites Ausführungsbeispiel einer Dialysemaschine gezeigt, bei welcher das zweite Ausführungsbeispiel einer Kassette zum Einsatz kommt. Das sich aus der Kombination des zweiten Ausführungsbeispiels einer Dialysemaschine und des zweiten Ausführungsbeispiels einer Kassette ergebende Dialysesystem ist dann in Figur 9 gezeigt.

Die beiden Ausführungsbeispiele unterscheiden sich dabei zum einen in der Ausgestaltung der Heizung, in der Kopplung zwischen der Dialysemaschine und der Kassette sowie in der Ausgestaltung der Aktoren und Sensoren.

### 2.1 Heizung

Das frische Dialysat muß auf Körpertemperatur gebracht werden, bevor es in den Bauchraum des Patienten befördert wird. Hierfür weist die Dialysemaschine eine entsprechende Heizung auf.

Die Heizung erfolgt dabei üblicherweise elektrisch über ein oder mehrere Heizelemente. Bei den Heizelementen kann es sich dabei z. B. um keramische Heizelemente handeln. Bei solchen keramischen Heizelementen ist eine Widerstandsbahn auf einem Keramikträger aufgebracht. Durch Anlegen einer Spannung an die Widerstandsbahn wird diese erhitzt, wodurch sich auch das keramische Trägermaterial erhitzt. Das keramische Heizelement ist dabei üblicherweise auf einer Heizplatte angeordnet. Diese kann beispielsweise aus Aluminium gefertigt sein. An die Heizplatte werden wiederum die Fluidwege angekoppelt, so daß das in den Fluidwegen befindliche Dialysat erhitzt werden kann.

Zum Erwärmen der Flüssigkeit stehen zwei unterschiedliche Ausgestaltungen zur Verfügung. Zum einen kann zunächst eine größere Menge an Dialysat erwärmt werden, welche erst nach der Aufwärmphase zum Patienten gepumpt wird. Dies erfolgt üblicherweise über einen Heizbeutel, welcher auf einer Heizplatte des Dialysegerätes aufliegt.

Bei dem Heizbeutel kann es sich dabei um den Dialysatbeutel handeln, in welchem das Dialysat zur Verfügung gestellt wird. Üblicherweise wird jedoch ein separater Heizbeutel eingesetzt, in welchem das Dialysat zum Aufheizen hineingepumpt wird. Ist das Dialysat im Heizbeutel erwärmt, wird es von dort aus zum Patienten gepumpt.

Ein solches Konzept ist bei den in Figuren 8 und 9 gezeigten zweiten Ausführungsbeispiel einer Dialysemaschine verwirklicht. Dabei ist ein Heizbeutel 67 vorgesehen, welcher auf einer Heizplatte 68 aufliegt. Die Heizplatte 68 ist dabei auf der Oberseite des Peritonealdialysegerätes angeordnet, so daß sie leicht zugänglich ist. Der Heizbeutel 67 ist dabei über eine Leitung 66' mit der Kassette verbunden. Die Kassette weist dabei die Ventile V5, V9 und V15 auf, über welche der Heizbeutel 67 mit den übrigen Komponenten des Fluidsystems verbunden werden kann. So kann frisches Dialysat von den Dialysatbehältern 10 über die Pumpkammern zum Heizbeutel 67 gepumpt werden. Zu Beginn einer Behandlung wird also zunächst der Heizbeutel 67 mit kaltem Dialysat gefüllt. Das Dialysat im Heizbeutel 67 wird dann über die Heizplatte 68 auf Körpertemperatur erwärmt. Daraufhin wird das Dialysat über die Pumpkammern zum Patienten gepumpt. Daraufhin kann der Heizbeutel 67 wieder befüllt werden, so daß die für den nächsten Behandlungszyklus benötigte Dialysatmenge erhitzt werden kann.

Vorteilhafterweise ist dabei im Bereich der Heizplatte 68 ein Temperatursensor 88 vorgesehen, welcher mit dem Heizbeutel 67 in Kontakt steht und so die Temperatur des Dialysats im Heizbeutel 67 messen kann. Weiterhin kann ein Temperatursensor an der Heizplatte oder am Heizelement vorgesehen sein, welcher die Temperatur des Heizelements oder der Heizplatte misst. Eine entsprechende Steuerung sorgt nun dafür, daß die Heizplatte nicht zu heiß für das Material des Beutels wird.

Der Heizbeutel 67 kann zudem Funktionen bei der Bilanzierung der Flüssigkeitsströme übernehmen. So kann die Heizplatte 68 Teil einer Waage 87 sein, über welche das Gewicht des Heizbeutels 67 bestimmbar ist. Hierdurch kann die Flüssigkeitsmenge, welche nach dem Erwärmen dem Patienten zugeführt wird, bestimmt werden.

Alternativ zu der beim zweiten Ausführungsbeispiel gezeigten Erwärmung des Dialysats über einen Heizbeutel kann das Dialysat auch erwärmt werden, während es zum Patienten gepumpt wird. Die Heizung arbeitet damit in Form eines Durchlauferhitzers, welcher das durch das Fluidsystem bewegte Dialysat erwärmt, während es durch die Fluidwege gepumpt wird.

Bei diesem Konzept ist ein Dialysatkanal vorgesehen, welcher an ein Heizelement der Dialysemaschine gekoppelt wird. Während das Dialysat durch den Dialysatkanal fließt, nimmt es dabei Wärme von dem Heizelement der Dialysemaschine auf.

Ein solches Konzept ist bei dem ersten Ausführungsbeispiel einer Dialysemaschine, welches in Figuren 6 und 7 gezeigt ist, implementiert. Dabei ist der Heizbereich in die Kassette integriert, wie dies bereits oben dargestellt wurde. Beim Ankoppeln der Kassette an die Dialysemaschine kommt dabei der Heizbereich der Kassette mit Heizelementen der Dialysemaschine thermisch in Kontakt.

Die Heizelemente können dabei ebenfalls als keramische Heizelemente ausgeführt sein und mit Heizplatten in Kontakt stehen, welche dann an den Heizbereich der Kassette gekoppelt werden. Wie bereits hinsichtlich der Kassette dargestellt, steht dabei sowohl mit der Oberseite als auch mit der Unterseite des Heizbereichs jeweils eine Heizplatte in Kontakt, welche das durch den Heizbereich fließende Dialysat erwärmt.

Am Zulauf und am Ablauf des Heizbereichs sind jeweils Temperatursensorbereiche in der Kassette vorgesehen, welche durch das Ankoppeln der Kassette mit Temperatursensoren der Peritonealdialyse in Kontakt kommen. Durch die Temperatursensoren T1 bis T3 kann so die Temperatur des in den Heizbereich einfließenden Dialysats sowie die Temperatur des aus dem Heizbereich herausfließenden Dialysats bestimmt werden. Weiterhin sind Temperatursensoren T4 und T5 vorgesehen, welche die Temperatur der Heizelemente und/oder der Heizplatten bestimmen.

Die Verwendung von mindestens zwei Heizelementen ermöglicht es dabei, die Heizelemente jeweils so zusammenzuschalten, daß sie bei einer Versorgungsspannung von 220 V im wesentlichen die gleiche Leistung abgeben wird wie bei einer Versorgungsspannung von 110 V. Hierfür werden die beiden Heizelemente bei 110 V in einer Parallelschaltung betrieben, während sie bei einer Versorgungsspannung von 220 V in einer Seriellschaltung betrieben werden. Eine solche Anpassung der Verschaltung der Heizelemente an die Versorgungsspannung kann dabei unabhängig davon, ob die Heizung gemäß dem ersten oder dem zweiten Ausführungsbeispiel erfolgt, implementiert werden.

### 2.2 Ankopplung der Kassette

Um eine Ankopplung der Aktoren und/oder Sensoren der Dialysemaschine an die entsprechenden Bereiche der Kassette zu ermöglichen, weist die Dialysemaschine eine Kassettenaufnahme mit einer Ankoppelfläche auf, an welcher die Kassette angekoppelt werden kann. An der Ankoppelfläche sind die entsprechenden Aktoren, Sensoren und/oder Heizelemente der Dialysemaschine angeordnet. Die Kassette wird mit dieser Ankoppelfläche so verpreßt, daß die entsprechenden Aktoren, Sensoren und/oder Heizelemente mit den entsprechenden Bereichen an der Kassette in Kontakt kommen.

Dabei ist vorteilhafterweise an der Ankoppelfläche der Dialysemaschine eine Matte aus einem flexiblen Material vorgesehen, insbesondere eine Silikonmatte. Diese sorgt dafür, daß die flexible Folie der Kassette mit den Stegbereichen der Kassette verpreßt wird und so die Fluidwege innerhalb der Kassette voneinander trennt.

Vorteilhafterweise ist weiterhin ein umlaufender Rand der Ankoppelfläche vorgesehen, welcher mit dem Randbereich der Kassette verpreßt wird. Vorteilhafterweise erfolgt die Verpressung dabei luftdicht, so daß zwischen der Ankoppelfläche und der Kassette ein Unterdruck aufgebaut werden kann.

Gegebenenfalls kann auch ein Vakuumsystem vorgesehen sein, welches Luft aus dem Raum zwischen Ankoppelfläche und Kassette abpumpen kann. Hierdurch wird eine besonders gute Ankopplung der Aktoren, Sensoren und/oder Heizelemente des Peritonealdialysegerätes mit den entsprechenden Bereichen der Kassette ermöglicht. Zudem erlaubt das Vakuumsystem eine Dichtigkeitsprüfung der Kassette. Hierfür wird nach dem Ankoppeln ein entsprechendes Vakuum angelegt und überprüft, ob dieses Vakuum aufrechterhalten wird.

Das Anpressen der Kassette erfolgt z. B. pneumatisch. Hierzu ist üblicherweise ein Luftkissen vorgesehen, welches mit Druckluft befüllt wird und so die Kassette an die Ankoppelfläche anpresst.

Die Kassettenaufnahme weist üblicherweise eine der Ankoppelfläche gegenüberliegende Aufnahmefläche auf, in welche das Hartteil der Kassette eingelegt wird. Die Aufnahmefläche weist hierfür vorteilhafterweise entsprechende Vertiefungen auf. Die Aufnahmefläche mit der eingelegten Kassette kann dann über eine pneumatische Anpressvorrichtung an die Ankoppelfläche angepresst werden.

Das Einlegen der Kassette kann dabei in unterschiedlicher Weise geschehen. Im ersten Ausführungsbeispiel einer Dialysemaschine, welches in Figur 6 gezeigt ist, ist hierfür eine Schublade 111 vorgesehen, welche aus der Dialysemaschine ausgefahren werden kann. In diese Schublade wird die Kassette eingelegt. Die Kassette wird dann zusammen mit der Schublade in die Dialysemaschine eingeschoben. Daraufhin erfolgt das Verpressen der Kassette mit der Ankoppelfläche, welche im Inneren des Gerätes angeordnet ist. Dabei werden Kassette und Koppelfläche zunächst mechanisch aneinander gefahren und dann pneumatisch miteinander verpreßt.

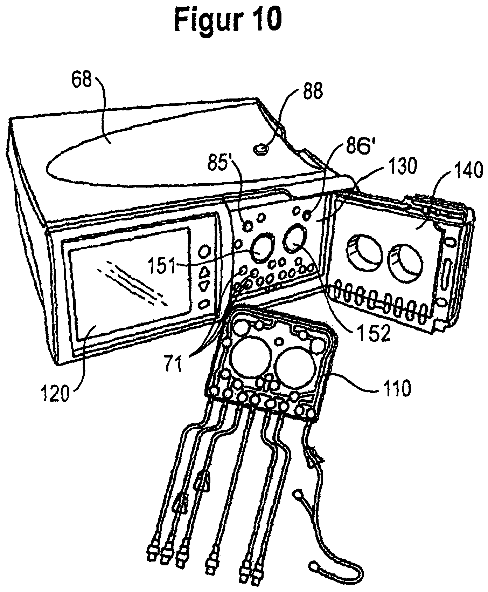

Das Ankoppeln einer Kassette 110 gemäß dem zweiten Ausführungsbeispiel ist in Figur 10 näher dargestellt. Die Ankoppelfläche 130 ist durch Öffnen einer Tür 140 frei zugänglich, so daß die Kassette in der richtigen Position an der Ankoppelfläche 130 angeordnet werden kann. Die Ankoppelfläche 130 ist dabei nach hinten zur Vertikalen geneigt, was ein leichteres Ankoppeln ermöglicht. Nun kann die Tür 140 geschlossen werden, so daß eine Aufnahmefläche an der Tür mit der Rückseite der Kassette in Kontakt kommt. Das Verpressen erfolgt nun durch ein an der Tür angeordnetes Luftkissen. Zudem wird ein Vakuum zwischen die Ankoppelfläche und die Kassette 110 angelegt.

Das erste Ausführungsbeispiel einer Dialysemaschine weist weiterhin eine Vorrichtung zum automatischen Konnektieren auf. Hierfür ist eine Konnektorenaufnahme 112 vorgesehen, in welche die Konnektoren der Dialysatbeutel 10 eingelegt werden. Die Konnektorenaufnahme 112 fährt dann in das Gerät hinein, wo ein Barcodeleser vorgesehen ist, welcher die auf den Konnektoren aufgebrachten Barcodes liest. So kann das Gerät überprüfen, ob die richtigen Beutel eingelegt wurden. Werden die richtigen Beutel erkannt, so fährt die Konnektoraufnahme 112 komplett ein und schließt so die Konnektoren der Beutel an den als Konnektoren ausgeführten Anschlüssen 11 der Kassette an.

Im zweiten Ausführungsbeispiel wurde auf eine solche automatische Konnektierung dagegen verzichtet. Daher sind an den Anschlüssen 11 der Kassette Schlauchabschnitte angeordnet, welche manuell über Konnektoren mit den entsprechenden Beuteln verbunden werden müssen.

### 2.3 Pumpaktoren

Das Pumpen der Flüssigkeit durch das Fluidsystem erfolgt in den Ausführungsbeispielen durch eine Membranpumpe, welche von den Pumpkammern 53 und 53' zusammen mit der flexiblen Folie der Kassette gebildet wird. Wird die flexible Folie dabei durch einen entsprechenden Pumpaktor in die Pumpkammer hineingedrückt, so wird Flüssigkeit aus der Pumpkammer in die geöffneten Bereiche der Fluidwege der Kassette gepumpt. Umgekehrt wird durch Herausziehen der Folie aus der Pumpkammer Fluid aus den Fluidwegen in die Pumpkammer gesaugt.

Der Pumphub erfolgt dabei durch Bewegen eines Pumpaktors in die Pumpkammer. Für den Saughub wird der Pumpaktor wieder von der Pumpkammer wegbewegt. Durch die luftdichte Verpressung von Kassette und Ankoppelfläche entsteht dabei ein Unterdruck, durch welchen die flexible Folie der Kassette dem Pumpaktor folgt und so wieder aus der Pumpkammer herausgezogen wird.

Um eine gute Ankopplung des Pumpaktors an die flexible Folie der Kassette zu ermöglichen, kann zudem ein Vakuumsystem vorgesehen sein. Über das Einstellen eines entsprechenden Vakuums zwischen der Ankoppelfläche und der Kassette kann dabei insbesondere die Kraft eingestellt werden, mit welcher die flexible Folie während eines Saughubs maximal von der Pumpkammer wegbewegt wird.

Hierdurch kann die Saugkraft der Pumpe sehr fein eingestellt werden. Die Pumpkraft wird dagegen durch die Schubkraft des Aktors eingestellt.

Die Bilanzierung der Flüssigkeitsströme kann dabei durch das Zählen der Saug- und Pumphübe erfolgen, da die Membranpumpe eine hohe Genauigkeit der mit jedem Hub gepumpten Flüssigkeitsmenge aufweist.

### 2.3.1. Hydraulischer Antrieb

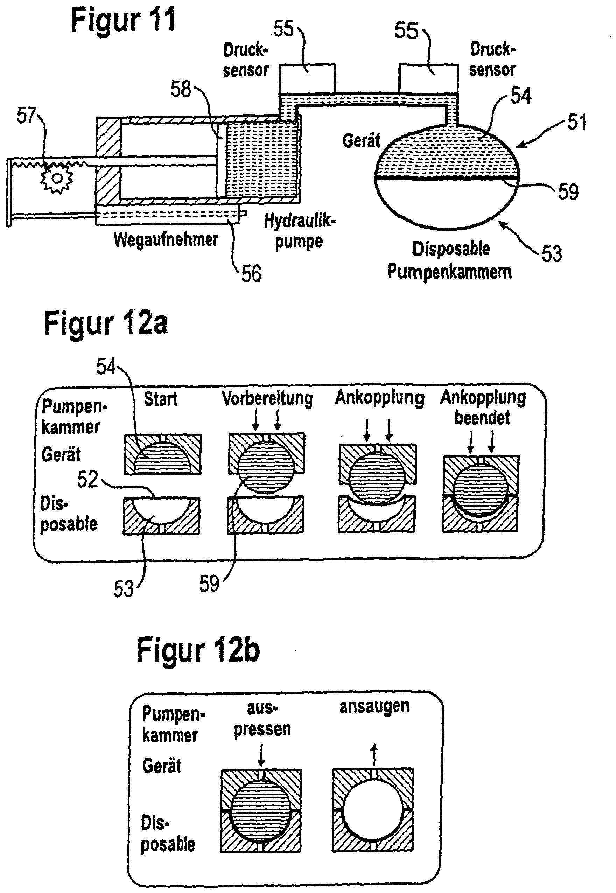

Der Aufbau eines ersten Ausführungsbeispiels eines Pumpaktors ist in Figur 11 gezeigt. Der Pumpaktor wird dabei hydraulisch bewegt. Hierfür ist eine Membran 59 vorgesehen, welche an der flexiblen Folie der Kassette anlegt. Die Membran 59 kann dabei z. B. aus Silikon gefertigt sein. Hinter der Membran 59 ist eine Kammer 54 vorgesehen, welche mit Hydraulikfluid gefüllt werden kann. Durch Anlegen eines Überdrucks in der Kammer 54 wird die Membran 59 und mit dieser die flexible Folie in die Pumpkammer 53 der Kassette hineingedrückt. Durch Anlegen eines Unterdruckes an die Kammer 54 wird die Membran 59 dagegen in die Kammer 54 hineingezogen. Durch den Unterdruck zwischen der flexiblen Folie und der Membran folgt die flexible Folie dieser Bewegung, so daß sich das Volumen der Pumpkammer 53 vergrößert. Der Pumpvorgang mit den Pumphub und dem Ansaughub ist dabei schematisch in Figur 12b dargestellt.

Zum Betrieb der Pumphydraulik ist eine Hydraulikpumpe 58 vorgesehen. Diese weist einen Zylinder auf, in welchem ein Kolben über einen Motor 57 hin und her bewegt werden kann. Hierdurch wird die Hydraulikflüssigkeit über eine entsprechende Verbindungsleitung in die Kammer 54 hineingepreßt oder aus dieser wieder herausgesogen. An der Hydraulikpumpe 58 ist dabei ein Wegaufnehmer 56 vorgesehen, über welchen die Bewegung des Kolbens aufgenommen werden kann. Hierdurch kann bestimmt werden, wieviel Hydraulikflüssigkeit in die Kammer 54 hineingepreßt bzw. wieviel Hydraulikflüssigkeit aus dieser entnommen wurde. Weiterhin sind Drucksensoren 55 an der Hydraulik vorgesehen, welche den Druck in dem Hydrauliksystem messen. Diese ermöglichen zum einen eine Funktionsüberprüfung der Hydraulik, da die Daten der Drucksensoren mit denen des Wegaufnehmers 56 verglichen werden können und hierdurch die Dichtigkeit des Hydrauliksystems überprüft werden kann.

Zudem ermöglichen die Drucksensoren eine Bestimmung des Drucks in der Pumpekammer 53 der Kassette. Wird die Hydraulikpumpe 58 nicht bewegt, so stellt sich ein Druckgleichgewicht zwischen der Kammer 54 und der Pumpkammer 53 ein. Der Druck der Hydraulikflüssigkeit entspricht damit dem Druck in der Pumpenkammer 53.

In Figur 12a ist nun der Ankoppelvorgang des Pumpenaktors an die Pumpenkammer 53 gezeigt. Zur Vorbereitung des Ankoppelns wird dabei zunächst die Kammer 54 mit Hydraulikfluid beaufschlagt, daß sich die Membran 59 nach außen wölbt. Daraufhin werden Ankoppelfläche und Kassette aufeinander zu bewegt, so daß die Membran 59 die flexible Folie der Kassette in die Pumpkammer 53 hineindrückt. Nach dem Verpressen von Ankoppelfläche und der Kassette ist der Raum zwischen der Membran und der flexiblen Folie nach außen luftdicht abgeschlossen, so daß die flexible Folie der Bewegung der Membran folgt. Dies ist in Figur 12b gezeigt.

Der in Figur 11 gezeigte Pumpaktor ist dabei beim ersten Ausführungsbeispiel einer Dialysemaschine implementiert, wie dies auch aus Figur 7 ersichtlich ist. Dabei ist für jede der beiden Pumpkammern 53 und 53' jeweils ein entsprechender Pumpaktor vorgesehen.

### 2.3.2 Elektromechanischer Antrieb

Alternativ kann der Pumpaktor auch elektromotorisch betrieben werden. Hierfür ist ein entsprechend geformter Stempel vorgesehen, welcher über einen Elektromotor, insbesondere über einen Schrittmotor gegen die flexible Folie gedrückt bzw. von dieser wegbewegt wird und so den Pump- bzw. Saughub erzeugt. Solche Pumpaktoren 151 und 152 sind bei dem Ausführungsbeispiel in Fig. 10 gezeigt. Vorteilhafterweise ist dabei ein Vakuumsystem vorgesehen, welches dafür sorgt, daß die flexible Folie dem Stempel auch bei der Saugbewegung folgt.

### 2.3.3 Pneumatischer Antrieb

Gemäß einer weiteren Alternative kann Pumpaktor auch pneumatisch bewegt werden. Dabei ergibt sich eine Betriebsweise, die weitgehend derjenigen gemäß der Beschreibung unter Punkt 2.3.1. entspricht.

### 2.4 Ventilaktoren

Als Ventilaktor kann ein Ventilstößel vorgesehen sein, welcher die flexible Folie der Kassette in eine entsprechende Kammer des Hartteils hineindrückt und so den Fluidkanal in diesem Bereich schließt. Der Ventilaktor kann dabei Z.B. pneumatisch betätigt werden. Der Stößel kann dabei über eine Feder vorgespannt sein, so daß er entweder drucklos öffnet oder drucklos schließt.

Alternativ kann der Ventilaktor über eine flexible Membran implementiert werden, welche hydraulisch oder pneumatisch bewegt wird. Die flexible Membran wird dabei durch Anlegen von Druck gegen die Kassette bewegt und drückt so einen entsprechenden Ventilbereich der flexiblen Folie in einen Fluidkanal, um diesen zu verschließen.

Ventilaktoren 71, welche an die Ventilbereiche V1 bis V16 der Kassette angekoppelt werden, sind in Fig. 10 auf der Ankoppelfläche erkennbar.

### 2.5 Sensoren

Die Dialysemaschine weist Sensoren auf, über welche die Maschine angesteuert bzw. deren ordnungsgemäßes Funktionieren überwacht werden kann.

Zum einen sind dabei ein oder mehrere Temperatursensoren vorgesehen, über welche die Temperatur des Dialysats und/oder der Heizelemente gemessen werden kann. Im ersten Ausführungsbeispiel sind die Temperatursensoren dabei an der Ankoppelfläche zur Kassette angeordnet und können so die Temperatur des durch die Kassette fließenden Dialysats messen. Im zweiten Ausführungsbeispiel ist dagegen ein Temperatursensor 88 auf der Heizplatte 68 vorgesehen, welcher die Temperatur des im Beutel 67 befindlichen Dialysats mißt. Weiterhin können an dem oder den Heizelementen Temperatursensoren vorgesehen sein.

Weiterhin können ein oder mehrere Drucksensoren vorgesehen sein, um den Druck in den Pumpkammern zu bestimmen. Hierdurch kann verhindert werden, das Dialysat mit zu hohem Druck zum Patienten gepumpt wird oder der Saugdruck beim Absaugen von Dialysat vom Patienten zu hoch wird.

Im ersten Ausführungsbeispiel erfolgt die Druckmessung dabei über Drucksensoren in der Hydraulik der Pumpaktoren, wie dies oben dargestellt wurde. Im zweiten Ausführungsbeispiel sind dagegen Drucksensoren 85' und 86' in der Ankoppelfläche vorgesehen, welche den Druck in entsprechenden Druckmessbereichen der Kassette direkt messen. Die Ankopplung dieser Drucksensoren an die Kassette wird dabei vorteilhafterweise durch ein Vakuumsystem sichergestellt.

### 2.6 Ein-/Ausgabeeinheit

Die Dialysemaschine umfaßt weiterhin eine Ein-/Ausgabeeinheit zur Kommunikation mit einem Bediener. Zur Ausgabe von Informationen ist dabei eine entsprechende Anzeige vorgesehen, welche z. B. durch Leuchtdioden, LCD-Anzeigen oder einen Bildschirm implementiert sein kann. Zur Eingabe von Befehlen sind entsprechende Eingabeelemente vorgesehen. Hierbei können z. B. Drucktasten und Schalter vorgesehen sein.

In beiden Ausführungsbeispielen ist dabei ein Touchscreen 120 vorgesehen, welcher eine interaktive Menüführung ermöglicht. Weiterhin sind Anzeigeelemente 121 und 122 vorgesehen, welche Zustände der Dialysemaschine kompakt darstellen.

Das erste Ausführungsbeispiel weist weiterhin einen Kartenleser 125 auf, über welchen eine Patientenkarte eingelesen werden kann. Auf der Patientenkarte können Daten zur Behandlung des jeweiligen Patienten abgespeichert sein. Hierdurch kann der Behandlungsablauf für den jeweiligen Patienten individuell festgelegt werden.

Die Peritonealdialyse weist weiterhin eine akustische Signaleinheit auf, über welche akustische Signale abgegeben werden können. Insbesondere kann dabei ein akustisches Warnsignal ausgegeben werden, wenn ein Fehlzustand registriert wird. Dabei ist vorteilhafterweise ein Lautsprecher vorgesehen, über welchen die akustischen Signale erzeugt werden können.

### 2.7 Steuerung

Die Peritonealdialyse weist weiterhin eine Steuerung auf, durch welche sämtliche Komponenten angesteuert und überwacht werden. Die Steuerung sorgt dabei für den automatischen Ablauf der Behandlung.

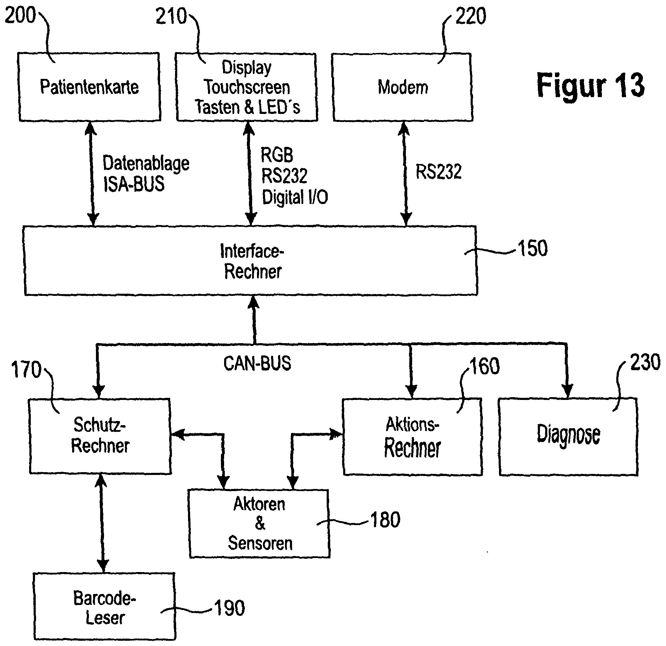

In Figur 13 ist nun der prinzipielle Aufbau eines Ausführungsbeispiels einer solchen Steuerung dargestellt.

Die Kommunikation mit dem Bediener sowie mit externen Informationsquellen erfolgt dabei über einen Interface-Rechner 150. Dieser kommuniziert mit einem Patientenkartenleser 200, einer Ein- und Ausgabeeinheit 210, welche der Kommunikation mit dem Patienten dient, sowie mit einem Modem 220. Über das Modem 220 kann z. B. eine aktualisierte Software aufgespielt werden.

Der Interface-Rechner 150 steht über einen internen Bus mit einem Aktionsrechner 160 und einem Schutzrechner 170 in Verbindung. Der Aktionsrechner 160 und der Schutzrechner 170 erzeugen eine Redundanz des Systems. Der Aktionsrechner 160 erhält dabei Signale von den Sensoren des Systems und berechnet die Steuersignale für die Aktoren 180. Der Schutzrechner 170 erhält ebenfalls Signale von den Sensoren 180 und überprüft, ob die vom Aktionsrechner 160 ausgegebenen Befehle korrekt sind. Stellt der Schutzrechner 170 einen Fehler fest, so leitet er eine entsprechende Notfallprozedur ein. Insbesondere kann der Schutzrechner 170 dabei ein Alarmsignal auslösen. Weiterhin kann der Schutzrechner 170 den Zugang zum Patienten schließen. Hierfür ist ein spezielles Ventil am patientenseitigen Ausgang der Kassette angeordnet, auf welches nur der Schutzrechner 170 Zugriff hat. Dieses Sicherheitsventil ist dabei im drucklosen Zustand geschlossen, so daß es bei einem Ausfall der Pneumatik automatisch schließt.

Der Schutzrechner 170 steht weiterhin mit dem Barcodeleser 190 in Verbindung, und überprüft so den Anschluss der korrekten Dialysatbeutel.

Weiterhin ist ein Diagnosesystem 230 vorgesehen, über welches Fehler des Systems ermittelt und behoben werden können.

### 3. Implementierung der Erfindung

Ein Ausführungsbeispiel der vorliegenden Erfindung, welches bei einem der oben dargestellten Dialysesysteme bzw. bei einer der oben dargestellten Dialysemaschinen zum Einsatz kommt, wird nun im folgenden dargestellt. Dabei kann das Ausführungsbeispiel der vorliegenden Erfindung mit einzelnen oder mehreren Komponenten, wie sie oben beschrieben wurden, kombiniert werden.

Wie eingangs erläutert, betrifft die vorliegende Erfindung eine Dialysemaschine mit einer Steuerung zur Einstellung eines kontinuierlichen Dialysatvolumenstroms, die grundsätzlich so aufgebaut ist, wie sie zuvor erläutert wurde. Im hier dargestellten Ausführungsbeispiel wird von einem hydraulischen Antrieb der Pumpaktoren 51 ausgegangen, wobei die Pumpaktoren natürlich auch pneumatisch angetrieben werden können. Gemäß der Erfindung wird ein kontinuierlicher Volumenstrom des Dialysats dadurch erreicht, daß der mittels dem in Figur 14 dargestellten Drucksensor ermittelte Hydraulikdruck P_{SollHyd} über eine konstante Energie für einen Pumpenhub bestimmt wird. Die Ansteuerung des Pumpenhubs mit konstanter Energie führt zu einer Volumenstrom-/Druckänderung im Gesamtsystem, die bewertet werden muß. Bei der Überschreitung eines maximal vorgegebenen Systemdrucks P_{PatMax} wird die Bewegung der Pumpe gestoppt. Da das Pump-Zeit-Volumen und die geförderte Menge bekannt sind, kann die konstante Energie für den nächsten Pumpenhub bestimmt werden. Aufgrund dieser Art der Ansteuerung ergeben sich folgende Eigenschaften des Sytems:
Da die Energie konstant eingestellt wird, wobei die eingestellte Energie der idealen Einstellung entspricht oder etwas kleiner als idealerweise benötigt eingestellt wird, besitzt das System die Eigenschaft auf Verschlüsse der Patientenzuleitungen oder auf bereits "entleerte" Patienten, d. h. Patienten gegen Ende des Dialysezyklus, derart zu reagieren, daß die Geschwindigkeit abnimmt, da die Last größer wird.

Wird dagegen die Energie etwas zu groß eingestellt, besitzt das System bei konstant eingestellter Energie die Eigenschaft, auf Verschlüsse der Patientenzuleitungen oder einen "entleerten" Patienten (gegen Ende des Zyklus) dadurch zu reagieren, daß die Last größer wird, während die Geschwindigkeit nahezu konstant bleibt. Dadurch übersteigt der Druck den zuvor festlegbaren Grenzdruck und die Pumpe stoppt.

Der Energiewert für den ersten Pumpenhub des Zyklus wird aus einer Schätzwerttabelle entnommen. Für jeden weiteren Kammerhub in diesem Zyklus wird die Energie unter Berücksichtigung des Pump-Zeit-Volumens, der geförderten Menge sowie der Überschreitung des Grenzdrucks P_{PatMax} adaptiert.

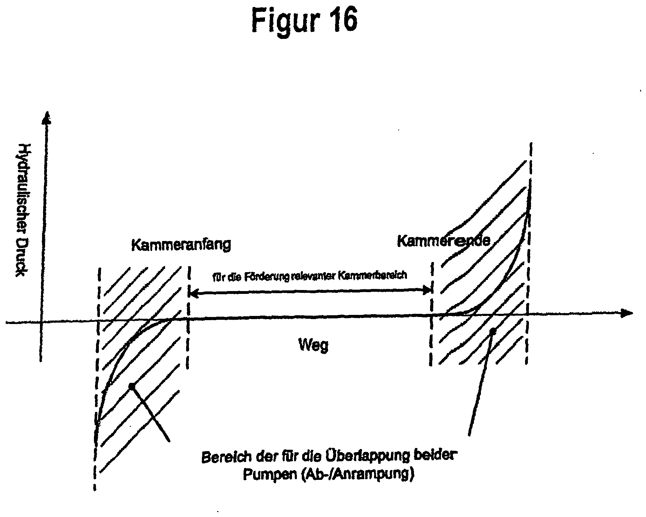

Bei Verwendung von Membranpumpen muß der Punkt der Membranspannung P_{Membran} ausgemessen werden, der das sogenannte Abrampen (d. h. den Gegendruck auf den hydraulischen Druck P_{Hyd}) des Flusses bezeichnet, wie die aus dem Diagramm in Figur 16 zu sehen ist. Dort ist der hydraulische Druck über dem Weg aufgetragen. Der Druckanstieg beim Abrampen ist im schraffierten Bereich des Diagramms, welcher mit Kammerende bezeichnet ist, dargestellt.

Gleichzeitig muß der Punkt der Membranspannung P_{Membran} ausgemessen werden, an dem keine Membranspannung P_{Membran} mehr wirkt. Dieser Bereich ist in Figur 16 mit dem schraffierten Bereich Kammeranfang bezeichnet.

Das erfindungsgemäße Verfahren führt dazu, daß wesentlich geringere Ansprüche an die Regelstrecke bzw. die Messsysteme zu stellen sind, als dies bisher der Fall war. Das führt zu einem gleichmäßigen Lauf der Hydraulikpumpe 58. Gleichzeitig können niedrige Volumenstromraten erreicht werden.

## Patentansprüche

1. Dialysemaschine mit mindestens zwei diskontinuierlichen Pumpen (51) und einer Steuerung zur Erzeugung eines kontinuierlichen Sollvolumenstromes des Dialysats, **dadurch gekennzeichnet, dass** die Steuerung die Energie zum Antrieb der Pumpen (51) mit einem entsprechend dem Pump-Zeit-Volumen der jeweiligen Pumpe (51) und dem geförderten Volumen bestimmten Wert konstant einstellt, wobei die konstante Energie für einen Pumpenhub bestimmt und zur Bestimmung der Energie eine konstante Leistung eingestellt wird.

2. Dialysemaschine nach Anspruch 1, **dadurch gekennzeichnet, dass** der Druck im geförderten Dialysat gemessen wird und, dass bei Überschreiten eines einstellbaren Druckgrenzwertes die Pumpen (51) angehalten werden.

3. Dialysemaschine nach Anspruch 1 oder 2, **dadurch gekennzeichnet, dass** die zur Versorgung der Pumpen (51) eingestellte Energie für jeden weiteren Pumpenhub in einem Pumpenzyklus unter Berücksichtigung des Pump-Zeit-Volumens, des geförderten Volumens sowie gegebenenfalls der Überschreitung des Druckgrenzwertes festgelegt wird.

4. Dialysemaschine nach einem der Ansprüche 1-3, **dadurch gekennzeichnet, dass** die zur Versorgung der Pumpen (51) eingestellte Energie für den ersten Pumpenhub als Schätzwert aus einer Schätzwerttabelle entnommen wird.

5. Dialysemaschine nach einem der Ansprüche 1-4, **dadurch gekennzeichnet, dass** bei Verwendung von Membranpumpen (51) die Membrancharakteristik berücksichtigt wird.

6. Dialysemaschine nach Anspruch 5, **dadurch gekennzeichnet, dass** der Punkt der Membranspannung bestimmt wird, bei dem die Membranpumpe (51) am Ende eines Pumphubes nicht mehr pumpt.

7. Dialysemaschine nach Anspruch 5 oder 6, **dadurch gekennzeichnet, dass** der Punkt der Membranspannung bestimmt wird, an der keine Membranspannung mehr wirkt.

8. Dialysemaschine nach einem der vorangegangenen Ansprüche , **dadurch gekennzeichnet, dass** die Steuereinheit Mittel zu Bestimmung der für einen Pumpenzyklus der Pumpen (51) einzusetzenden Energie umfasst und die Energie zum Antrieb der Pumpen (51) für einen Pumpenhub mit einem entsprechend dem Pump-Zeit-Volumen der jeweiligen Pumpe (51) und dem geförderten Volumen bestimmten Wert konstant einstellt.

9. Dialysemaschine nach Anspruch 8, **dadurch gekennzeichnet, dass** die diskontinuierlichen Pumpen Membranpumpen (51) sind.

## Claims

1. A dialysis machine with at least two discontinuous pumps (51) and a controller for generating a continuous desired volume flow of the dialysate, **characterized in that** the controller sets the energy for driving the pumps (51) to be constant with a value determined corresponding to the pump-time volume of the respective pump (51) and the delivered volume, wherein the constant energy is determined for a pump stroke, and a constant power is set for determining the energy.

2. The dialysis machine according to claim 1, **characterized in that** the pressure in the delivered dialysate is measured and that upon exceedance of an adjustable pressure limit value the pumps (51) are stopped.

3. The dialysis machine according to claim 1 or 2, **characterized in that** the energy set for supplying the pumps (51) is determined for each further pump stroke in a pump cycle by taking account of the pump-time volume, the delivered volume and possibly the exceedance of the pressure limit value.

4. The dialysis machine according to any of claims 1- 3, **characterized in that** the energy set for supplying the pumps (51) for the first pump stroke is taken as an estimate from an estimate table.

5. The dialysis machine according to any of claims 1- 4, **characterized in that** when using diaphragm pumps (51) the diaphragm characteristic is taken into account.

6. The dialysis machine according to claim 5, **characterized in that** the point of the diaphragm tension is determined, at which the diaphragm pump (51) no longer pumps at the end of a pump stroke.

7. The dialysis machine according to claim 5 or 6, **characterized in that** the point of the diaphragm tension is determined at which no more diaphragm tension acts.

8. The dialysis machine according to any of the preceding claims, **characterized in that** the control unit comprises means for determining the energy to be used for a pump cycle of the pumps (51) and sets the energy for driving the pumps (51) to be constant for a pump stroke with a value determined corresponding to the pump-time volume of the respective pump (51) and the delivered volume.

9. The dialysis machine according to claim 8, **characterized in that** the discontinuous pumps are diaphragm pumps (51).

## Revendications

1. Machine de dialyse avec au moins deux pompes discontinues (51) et un contrôleur pour générer un débit volumique continu souhaité du dialysat, **caractérisée en ce que** le contrôleur règle l'énergie pour l'entraînement des pompes (51) à une valeur constante déterminée en fonction du volume de temps de pompage de la pompe respective (51) et du volume délivré, l'énergie constante étant déterminée pour une course de pompe et une puissance constante étant réglée pour la détermination de l'énergie.

2. Machine de dialyse selon la revendication 1, **caractérisée en ce que** la pression dans le dialysat délivré est mesurée et **en ce que** les pompes (51) sont arrêtées lorsqu'une valeur limite de pression réglable est dépassée.

3. Machine de dialyse selon la revendication 1 ou 2, **caractérisée en ce que** l'énergie réglée pour l'alimentation des pompes (51) est déterminée pour chaque course de pompe supplémentaire dans un cycle de pompe, en tenant compte du volume de temps de pompage, du volume délivré et, le cas échéant, du dépassement de la valeur limite de pression.

4. Machine de dialyse selon l'une quelconque des revendications 1-3, **caractérisée en ce que** l'énergie réglée pour l'alimentation des pompes (51) pour la première course de pompe est tiré comme valeur estimée d'un tableau de valeurs estimées.

5. Machine de dialyse selon l'une quelconque des revendications 1-4, **caractérisée en ce que**, lorsque des pompes à membrane (51) sont utilisées, la caractéristique de membrane est prise en compte.

6. Machine de dialyse selon la revendication 5, **caractérisée en ce que** le point de la tension de membrane, auquel la pompe à membrane (51) cesse de pomper à la fin d'une course de pompage, est déterminé.

7. Machine de dialyse selon la revendication 5 ou 6, **caractérisée en ce que** le point de la tension de membrane, auquel aucune tension de membrane n'agit plus, est déterminé.

8. Machine de dialyse selon l'une quelconque des revendications précédentes, **caractérisée en ce que** l'unité de commande comprend des moyens pour déterminer l'énergie à utiliser pour un cycle de pompage des pompes (51) et règle l'énergie pour l'entraînement des pompes (51) pour une course de pompe à une valeur constante déterminée en fonction du volume de temps de pompage de la pompe respective (51) et du volume délivré.

9. Machine de dialyse selon la revendication 8, **caractérisée en ce que** lesdites pompes discontinues sont des pompes à membrane (51).
